# EUROPEAN PATENT SPECIFICATION

(11) **EP 1 515 774 B1**
(45) Date of publication and mention of the grant of the patent: **20.06.2012**
(21) Application number: 02792533.8
(22) Date of filing: 27.12.2002
(51) Int. Cl.: A61F 5/08, A61B 5/087

(54) **NASAL DEVICES INCLUDING DILATION AND USER COMMUNICATION**
NASENVORRICHTUNGEN, EINSCHLIESSLICH DILATATION UND BENUTZERKOMMUNIKATION
DISPOSITIFS POUR LE NEZ PERMETTANT LA DILATATION D'UN TISSU ET L'ETABLISSEMENT D'UNE COMMUNICATION AVEC L'UTILISATEUR

(30) Priority: 31.12.2001 US 346071 P
(43) Date of publication of application: 23.03.2005
(73) Proprietor: CNS, INC., Minneapolis, MN 55439-0802 (US)
(72) Inventor: FENTON, Gustav, R., Minneapolis, MN 55409 (US)
(74) Representative: Walker, Ralph Francis
(86) International application number: PCT/US2002/041505
(87) International publication number: WO 2003/057304

(56) References cited:
- WO-A-96/01093
- DE-A1- 10 152 614
- JP-A- 10 120 555
- US-A- 5 413 111
- US-A- 5 611 333
- US-A- 5 706 800
- US-A- 5 947 119

## Description

### FIELD OF THE INVENTION

The present invention relates to apparatus for influence surface tissue for therapeutic and/or aesthetic reasons. In particular, the present invention is directed to discrete embodiments and techniques for sensing, signaling, and/or dilating tissue proximate a nasal passage using an external device.

### BACKGROUND OF THE INVENTION

The field of endeavor related to dilation of nasal passages and adjacent tissue using over the nose-type dilator devices has a short and active history. One active participant and innovator in this field is the present assignee, CNS, Inc. (CNS) of Eden Prairie, MN.

The disposable over the nose dilator devices of the prior art provide sufficient albeit rudimentary dilation of tissue adjacent nasal passageways and thus provide a modicum of increased respiration and relief from snoring in the vast majority of users. However, these prior art designs are generally not adjustable by a user, and do not incorporate additional functionality for, or generate additional benefits to, the user. For example US-A-5,947,119 discloses a nasal air flow control device, insertable in the user's nose.

Thus, a need in the art exists for continued innovation and greater functionality for nasal devices. For example, a need exists for dilator devices that are simple to fabricate, that effectively dilate tissue, that may be adjusted in length and magnitude of lifting force imparted to the tissue, that may be accurately aligned relative to the tissue, that are more easily removed from the tissue, that are reusable and which are, in general, more comfortable to the user than prior art dilator devices. Additionally, a need exists for a nasal-mounted device having a signal unit for detecting nasal vibrations or other signals indicative of physiological functions of the user. The signal unit may provide biofeedback to a user to assist in the control of breathing to, for example, assist in control or prevention of panic attacks, facilitate meditations, etc. DE-A-101 52 614 discloses a device which can be attached to a user's nose and incorporates a sensor to detect information about the user's breathing.

The present invention provides a nasal device according to claim 1. The present invention builds upon prior commercial embodiments of tissue dilator devices and addresses several still unmet needs in the art of fabricating, aligning, adjusting, applying, using, re-using and/or removing nasal tissue devices.

On particular embodiment disclosed herein provides a nasal device having a flexible strip of material adapted to be adhesively secured on nasal surfaces of a user and to provide tactile communication to the user at the nasal surfaces. One embodiment of the invention may include a sensor for detecting a plurality of physiological parameters of the user. In an embodiment of the present invention the tactile communication may be controlled in response to the sensed physiological information. In an embodiment of the present invention, an acoustic sensor is used to sense vibrations of the nasal surfaces related to the user's breathing pattern. The present includes multiple sensors and multiple means to communicate to the user via the nasal surface.

The several embodiments of the present invention are described with reference to examples of forms of the invention comprehended by the devices taught, enabled, described, illustrated and claimed herein but all structures and methods which embody similar functionality are intended to be covered hereby. These embodiments include without limitation the following numbered, discrete forms of the invention, as more fully developed in the detailed description appearing hereinbelow.

### BRIEF DESCRIPTION OF THE DRAWINGS

In the drawings which accompany this disclosure, like elements are referred to with common reference numerals. The drawings are not rendered to scale and illustrate only a few of the many, many embodiments of tissue dilators which may be created according to the teaching of the present invention.
FIG. 1 is perspective view of the embodiment of the present invention shown as placed upon nasal surfaces of a user.
FIG. 2 is a perspective view of an embodiment of a nasal dilator according to the present invention wherein at least one signal unit is integrated into said nasal dilator either at an adhesive pad location or in the base member of said nasal dilator.
FIG. 3 is a simplified depiction of one type of signal unit which may be incorporated into a nasal surface contacting device.
FIG. 4 is a plan view of an embodiment of the present invention having two opposing pocket features formed in an upper surface of a nasal dilator device.
FIG. 5 is an elevational side view in cross section taken along the lines 5-5 of FIG. 4 of the embodiment of the nasal dilator depicted in FIG. 4.
FIG. 6 depicts an elevational side view in cross section of another embodiment of the present invention depicted in FIG. 4 and FIG. 5 wherein two opposing pocket features are each separate pieces spaced apart and adapted to receive at least one resilient member (shown in FIG. 7).
FIG. 7 is a perspective view of several resilient members usable with the present invention.

### DETAILED DESCRIPTION OF THE PREFERRED EMBODIMENTS

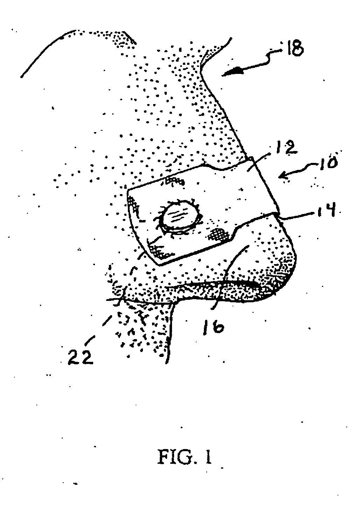

FIG. 1 illustrates in perspective view one embodiment of a nasal device 10 according to the present invention. Nasal device 10 includes a strip of flexible material 12 and an adhesive 14 for securing nasal device 10 to nasal surfaces 16 of a user 18. A resilient member 20 may be provided to create lift to the nasal surfaces 16. Other embodiments of the present invention may not require resilient means 20 to provide lift to the nasal surfaces 16. Nasal device 10 further includes a signal unit 22 for providing communication to user 18 during usage of device 10.

Particular selection of a flexible material 12, adhesive 14, and resilient member 20 would be appreciated by those skilled in the art, particularly with reference to U.S. Patents 5,476,091; 5,533,499; 5,494,103; 5,653,224; 6,318,362; 6,196,228; 6,354,436; 5,546,929; 5,553,605; 5,718,224; and 5,479,944, each patent being incorporated by reference herein for all purposes.

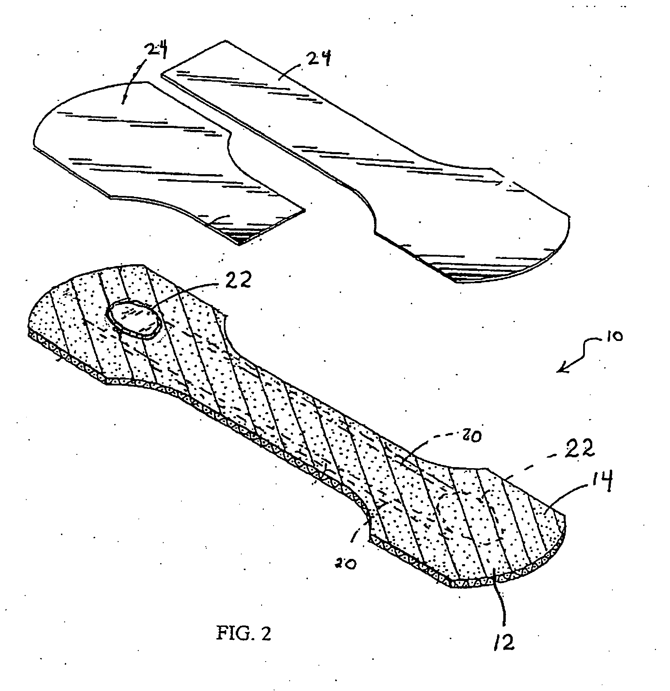

Now with reference to FIG. 2 which is a perspective view of an embodiment of a nasal dilator 10 according to the present invention wherein at least two signal units 22 is integrated with a nasal dilator 10. Signal units 22 may be disposed at other locations, and multiple signal units 22 may be utilized. Two or more of the signal units 22 may be affixed to the dilator 10. For example, a signal unit 22 may be disposed at or near a midpoint of the dilator 10 so that firm contact is established with the bridge of the nose of the user and another may be disposed in a location surrounded by, or nearly surrounded by, adhesive material. Signal units 22 may be incorporated with dilator 10 at the time of manufacture or may be a removable device which is reusable by the user.

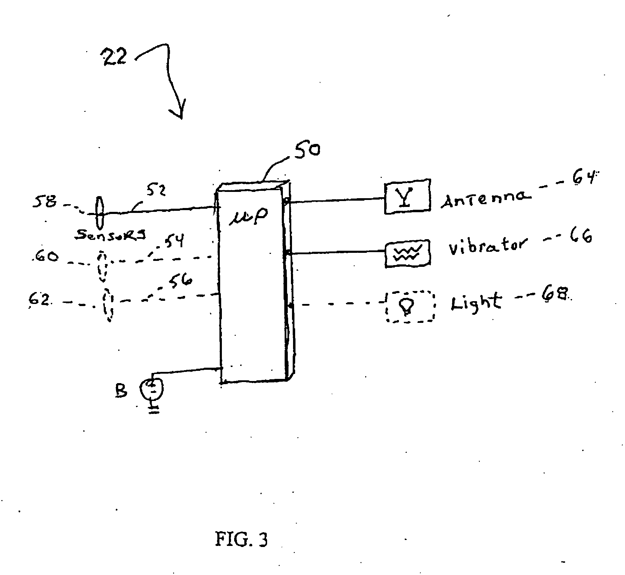

Release liners 24 may be removed to expose adhesive 14. Referring now to FIG. 3, signal units 22 may include a microprocessor 50 which receives signals 52, 54, 56 from two or more sensors 58, 60, 62 and controls operation of functions such as communication, mechanical vibration, light emission, etc. Sensor 58 may be an acoustic sensor for sensing vibrations of nasal tissue indicative of a user's breathing patterns. A variety of known acoustic sensor may be utilized, for example, acoustic sensor may be piezoelectric device, etc. Two or more sensor may be provided. Other sensor technologies may also be applicable. Microprocessor 50 may collect signal data from sensors 58, 60, 62 and perform data computations to control two or more outputs 64, 66, 68. Microprocessor 50 may include a memory means for storing collected data from nasal sensors 58, 60, 62. Microprocessor 50 may control a wireless communication device 64 including a transceiver and an antenna for communicating information to a user or another. Communication device 64 may receive or transmit via radio-frequency, infrared, or light-based communication. Communication device 64 may transmit a signal which is received by another antenna and subsequently processed by an external data processor. Communication unit 64 may also provide for signal reception to control a function of microprocessor 50. Communication device 64 may be integrated with microprocessor 50. Microprocessor 50 may also control operation of a mechanical vibrator 66 to signal a user. Mechanical vibrator 66 may be a piezoelectric device, for example. The function of vibrator 66 is to produce tactile and/or acoustic communication to a user of the nasal device. Vibrator 66 may apply a periodic pulse signal which is detectable by the user and which may be inaudible. For example, such an inaudible tactile pulse may be detected simply due to the contact between nasal tissue 46 and signal unit 22. In another embodiment, vibrator 66 may also produce an audible signal. Vibrator 66 may simply transmit a small impulse or series of impulses to a user, and not otherwise vibrate for a period of time. In this sense, vibrator 66 may or may not transmit a cyclic response to user.

Microprocessor 50 may control vibrator 66 to provide a "pacing" function which may help a user relax or compare their own present rate of respiration to a referenced respiration rate; for example during a panic attack or hyperventilation. Microprocessor 50 may function to receive an input corresponding to the current respiration rate and output a signal to the user via vibrator 66 indicative of a desired respiration rate (which may be higher or lower than the current rate) by reference to a look-up table or by other calculations, etc. The signal units 22 may also help a user meditate or increase a level of concentration due to application of rhythmic pulses. In a related embodiment, the rate of the pulses produced by the vibrator 66 may be adjusted by the user to an increased tempo to promote a greater rate of respiration or movement of the user. Such an adjustable timing function may also be used to simply allow the user to set a comfortable pace, so that, for example, when the user is engaged is competition or sports events the user can sense when the difference between a quiescent state and an excited state and may more rapidly transition from one state to the other state. In another embodiment, signal units 22 may detect voice commands of the user to control operation of vibrator 66 or other user signaling means. Signal units 22 receive additional signals from sensors 60, 62. For example, other physiological conditions such as a heart rate parameter, a blood pressure parameter, or a temperature parameter may be detected and utilized alone or in conjunction with breathing information as inputs to microprocessor 50. Signal unit 22 may also control light device 68 for communicating information related the input signals 52, 54, 56 to the user.

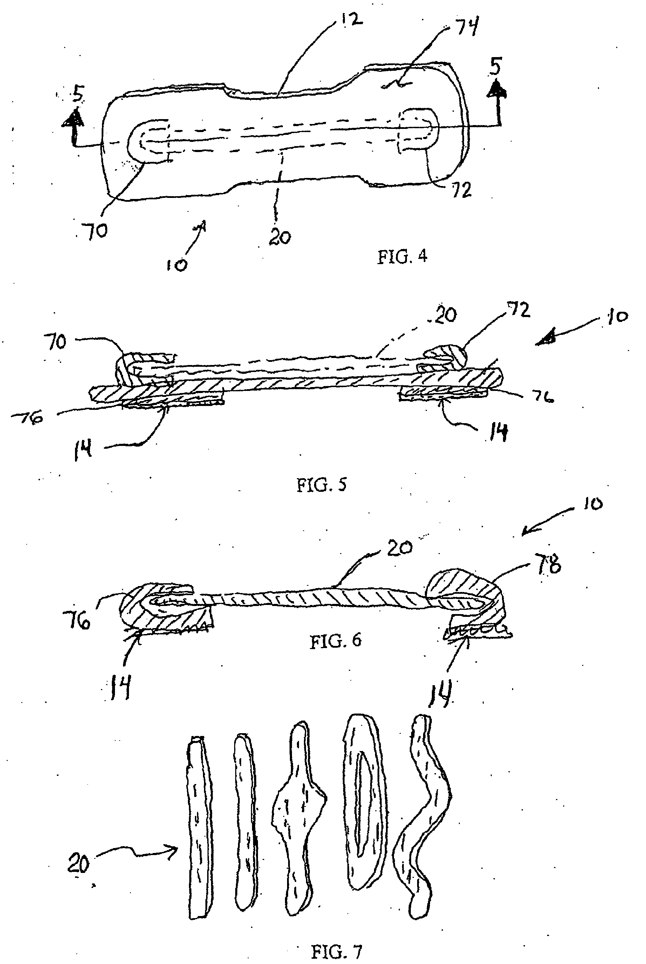

Referring now to FIG. 4, which is a plan view of an embodiment of a nasal dilator 10 of the present invention which comprises an elongated base member of flexible material 12 having two opposing pocket features 70, 72 formed in an upper major surface 74 of the nasal dilator 10. A pair of patches, or a layer, of adhesive 14 (shown in FIG. 5 and FIG. 6) is disposed on a lower surface to adhesively couple the dilator 10 to tissue of a user (not shown). Pockets 70, 72 are configured to receive at least one resilient member 20 (shown in ghost in FIG. 4) which essentially comprise an elongated member composed of a material which generates a restoring force. The restoring force tends to resist such bending so that when the ends of the resilient member 20 are constrained in the pockets 70, 72 tissue underlying the adhesive 14 receives said restoring force as a lifting force more or less directed orthogonal to the surface of the tissue to promote expansion of underlying nasal passages.

As shown in FIG. 5, which is an elevational side view in cross section taken along the lines 5 - 5 of FIG. 4, the pockets 70, 72 may have a pair of openings facing each other or may comprise an open-ended strap feature which captures the lifting force provided by the resilient member 20 so that the pads 76 which are adhered to the tissue imparts substantially all said lifting force orthongonally to said tissue. In the embodiment depicted in FIG. 4 and FIG. 5 the pockets 70, 72 appear as individual parts vis-à-vis the base 12 and the pads 76 but these parts may be fabricated as a single integral unit of construction. This form of the invention is inherently at least partially reusable in that the resilient member 20 may be retrieved from the assembly and used with another structure having the combination of a base 12, pockets 70, 72 and pads 76.

FIG. 6 depicts an elevational side view in cross section of form of the present invention depicted in FIG. 4 and FIG. 5 except that the two opposing pocket features are each separate parts spaced apart, supported on the tissue and adapted to receive at least one resilient member 20. In fact, this special form of the present invention is an embodiment having two discrete structures which each corresponding only to a single pocket and pad pair and wherein a resilient member 20 bridges between them. One advantage to forms of the present invention such as these is not only the ability to reuse the resilient member 20, but also that the resilient member 20 may be selected from a plurality of resilient members 20 each providing a slightly different magnitude of lifting force or configuration.

As illustrated in FIG. 7 is a plan view of several different resilient members 20 usable according to the present invention. The resilient members 20 may be used individually or combined with other of said resilient members 20 as desired. In addition to the shapes depicted the resilient members may have an undulating or serpentine cross sectional shape (e.g., analogous to the letters "W" or "U" and the like) . In addition, a resilient member 20 usable with most forms of the present invention forms a geometric shape in lateral cross section and includes internally hollow and perforated forms of such resilient members 20, so long as the resilient member 20 so constructed provides the required lifting force to the tissue. Furthermore, in several adjustable-length embodiments of the present invention, resilient members 20 of all shapes may advantageously be partially perforated and manually shortened to suit a particular desired length or cut with an implement such as a scissor, knife or other sharpened blade and the like.

In a further related embodiment, a single resilient member is disposed in a pocket or sleeve or the dilator and the resilient member essentially floats in said pocket or sleeve when the body of the dilator is twisted and thus the resilient member does not twist. Preferably, such a single resilient member is elongated and has a cross section that is round and the resilient member may comprise a hollow tube. Of course the resilient member may have diverse symmetric and asymmetric cross sectional shapes, including having two or more major planar surfaces.

Another embodiment of dilator devices according to the present invention include a family of "pacing" dilator devices. That is, dilator devices that function somewhat similarly to a metronome providing biofeedback to the user related to the rhythm and rate of respiration. This family of dilator devices has many forms, but one preferred form involves use of a substantially flat elongated resilient member that remains substantially flat after being applied to dilate tissue on opposing sides the nose of a user. In this form of the present invention, the dilator device is constructed to emit a vibration in response to breathing conditions of the user. Thus, in the one preferred embodiment, the vibration is emitted by the resilient member when the user inhales or exhales, thus slightly distorting the resilient member from its substantially flat configuration. The noise may be audible or may be passively transmitted through the bone structure of the user and sensed as a slight vibration originating near the nose of the user. An alternate form of this invention implements a hinged resilient member - having a small range of motion for said hinge and preferably including a detent and a corresponding boss member- so that when the hinge is activated, the boss enters the detent (and "clicks") and then exits the hinge (providing another "click"). Another form of this invention uses an elongated resilient member having opposing major planar surfaces that is bowed due to compression forces acting on the ends of the resilient member. The effect is similar to a batten constrained in a pocket of a sail on a sailboat. That is, each time a force impinges upon the convex side of the resilient member of a first state, the resilient member "snaps" to the opposite configuration of a second state (i.e., convex side becomes concave side and vice versa). When balanced with a relatively weak compressive force, the resilient member reciprocates between the first state and second state. The preferred form of this embodiment of the invention thus provides biofeedback to the user as the resilient member transitions from the first state to the second state. The resilient member may be adhered to the bridge of a user's nose or simply affixed to the user's nose thus forming a pivot location at the bridge of the nose and wherein each end of the resilient member is coupled to the tissue via a length of generally non-elastic material having an attachment coupling at the end. The attachment coupling may comprise a suction cup, a patch of adhesive or adhesive disposed upon the interior cavity of said suction cup and the like. Of course, the use of a resilient member that is maintained in a state of compression may be used without practicing the "pacing" function described above, as such a resilient member will still provide the required lifting forces to the tissue regardless whether or not the resilient member transitions between two energy states.

## Claims

1. A nasal device (10) for communicating physiologically- related information to a user, said device comprising a flexible strip (12) of material having a first end region and a second end region and an intermediate region, said flexible strip (12) adapted to be adhesively secured to nasal surfaces (16) of a user, and ***characterised* by**:
a signal device (22) which includes a plurality of sensors (58, 60, 62) which sense physiological information of the user while disposed upon the nasal surfaces (16) and includes a plurality of communication means (64) which communicate information to the user related to sensed physiological information via one or more of acoustic vibrations, tactile contacts and light emissions.

2. The nasal device (10) of claim 1 ***characterised* in that** the signal device (22) is disposed at the first end region, the second end region, or the intermediate region.

3. The nasal device (10) of claim 1 ***characterised* in that** the signal device (22) receives information from an external source and utilizes this information to alter an output process.

4. The nasal device (10) of claim 1 ***characterised* in that** the signal device (22) transmits information to a remote transceiver for subsequent signal processing.

5. The nasal device (10) of claim 4 ***characterised* in that** the signal device (22) transmits via radio-frequency or infra red communication.

6. The nasal device (10) of claim 1 ***characterised* in that** the signal device (22) includes memory means for storing information.

7. The nasal device (10) of claim 1 ***characterised* in that** the signal device (22) includes a timing means for controlling application of a tactile contact.

8. The nasal device (10) of claim 7 ***characterised* in that** tactile contact is a periodic vibration.

9. The nasal device (10) of claim 1 ***characterised* in that** the signal device (22) is integrated within flexible strips (12) of material.

10. A nasal device (10) of claim 1 ***characterised* by** further comprising a resilient member coupled to the flexible strip (12) which provides a lifting force to portions of the nasal surfaces (16) of the user.

11. A nasal device (10) according to claim 1 ***characterised* by** an adhesive layer (14) coupled to the flexible strip (12) of material for securing the flexible strip to the nasal surfaces (16), and a tactile vibration means (66) for communicating tactile information to a user at the nasal surfaces (16), said tactile information being related to the physiological information.

12. The nasal device (10) of claim 11 ***characterised* in that** the sensor means includes an acoustic sensor for sensing vibrations of the nasal surfaces (16) related to the user's breathing pattern.

13. The nasal device (10) of claim 11 ***characterised* by** a resilient member coupled to the flexible strip of material which provides a lifting force to the nasal surfaces (16) of the user.

14. A nasal device (10) according to claim 11 ***characterised* in that** the tactile vibration means (66) provides a periodic tactile transmission to the nasal surfaces (16) of the user.

15. A nasal device (10) according to claim 14 ***characterised* by** further comprising a microprocessor (60) for receiving a signal from a sensor (58, 60, 62) and for controlling a function of the tactile vibration means (66).

## Patentansprüche

1. Nasenvorrichtung (10) zum Übertragen von physiologiebezogenen Informationen an einen Benutzer, mit einem flexiblen Materialstreifen (12), der einen ersten Endbereich und einen zweiten Endbereich und einen Zwischenbereich aufweist, wobei der flexible Streifen (12) angepasst ist, haftend an Nasenflächen (16) eines Benutzers gesichert zu werden und **gekennzeichnet ist durch**:
eine Signalvorrichtung (22), die eine Vielzahl von Sensoren (58, 60, 62) aufweist, die während auf den Nasenflächen (16) angeordnet physiologische Informationen des Benutzers erfassen, und eine Vielzahl von Kommunikationsmitteln (64) aufweist, die Informationen zu dem Benutzer übertragen, die sich auf erfasste physiologische Informationen beziehen, und zwar über akustische Vibrationen, taktile Kontakte und/oder Lichtemissionen.

2. Nasenvorrichtung (10) nach Anspruch 1, **dadurch gekennzeichnet, dass** die Signalvorrichtung (22) an dem ersten Endbereich, dem zweiten Endbereich oder dem Zwischenbereich angeordnet ist.

3. Nasenvorrichtung (10) nach Anspruch 1, **dadurch gekennzeichnet, dass** die Signalvorrichtung (22) Informationen von einer externen Quelle empfängt und diese Informationen verwendet, um einen Ausgabeprozess zu verändern.

4. Nasenvorrichtung (10) nach Anspruch 1, **dadurch gekennzeichnet, dass** die Signalvorrichtung (22) Informationen zu einem Fern-Transceiver zur nachfolgenden Signalverarbeitung überträgt.

5. Nasenvorrichtung (10) nach Anspruch 4, **dadurch gekennzeichnet, dass** die Signalvorrichtung (22) über Hochfrequenz- oder Infrarotkommunikation überträgt.

6. Nasenvorrichtung (10) nach Anspruch 1, **dadurch gekennzeichnet, dass** die Signalvorrichtung (22) ein Speichermittel zum Speichern von Informationen aufweist.

7. Nasenvorrichtung (10) nach Anspruch 1, **dadurch gekennzeichnet, dass** die Signalvorrichtung (22) ein Zeitzählungsmittel zum Kontrollieren des Anwendens eines taktilen Kontakts aufweist.

8. Nasenvorrichtung (10) nach Anspruch 7, **dadurch gekennzeichnet, dass** der taktile Kontakt eine periodische Vibration ist.

9. Nasenvorrichtung (10) nach Anspruch 1, **dadurch gekennzeichnet, dass** die Signalvorrichtung (22) in dem flexiblen Materialstreifen (12) integriert ist.

10. Nasenvorrichtung (10) nach Anspruch 1, des Weiteren **gekennzeichnet durch** ein mit dem flexiblen Streifen (12) verbundenes federndes Element, das an Abschnitten der Nasenflächen (16) des Benutzers eine hebende Kraft bereitstellt.

11. Nasenvorrichtung (10) nach Anspruch 1, **gekennzeichnet durch** eine mit dem flexiblen Materialstreifen (12) verbundene Haftschicht (14) zum Sichern des flexiblen Streifens an den Nasenflächen (16) und ein taktiles Vibrationsmittel (66) zum Übermitteln einer taktilen Information an den Nasenflächen (16) zu einem Benutzer, wobei die taktile Information mit der physiologischen Information in Zusammenhang steht.

12. Nasenvorrichtung (10) nach Anspruch 11, **dadurch gekennzeichnet, dass** das Sensormittel einen Akustiksensor zum Erfassen von Vibrationen der Nasenflächen (16) aufweist, die mit dem Atmungsmuster des Benutzers in Zusammenhang stehen.

13. Nasenvorrichtung (10) nach Anspruch 11, **gekennzeichnet durch** ein Federelement, das mit dem flexiblem Materialstreifen verbunden ist und das an den Nasenflächen (16) des Benutzers eine hebende Kraft bereitstellt.

14. Nasenvorrichtung (10) nach Anspruch 11, **dadurch gekennzeichnet, dass** das taktile Vibrationsmittel (66) eine periodische taktile Übertragung an die Nasenflächen (16) des Benutzers bereitstellt.

15. Nasenvorrichtung (10) nach Anspruch 14, **gekennzeichnet durch** einen Mikroprozessor (60) zum Empfangen eines Signals von einem Sensor (58, 60, 62) und zum Steuern einer Funktion des taktilen Vibrationsmittels (66).

## Revendications

1. Dispositif nasal (10) permettant de communiquer des informations de nature physiologique à un utilisateur, ledit dispositif comprenant une bande flexible (12) de matériau ayant une première zone d'extrémité, une seconde zone d'extrémité et une zone intermédiaire, ladite bande flexible (12) étant adaptée pour être fixée de manière adhésive à des surfaces nasales (16) d'un utilisateur, et **caractérisé par** :
un dispositif de signal (22) comprenant une pluralité de capteurs (58, 60, 62) qui détectent les informations physiologiques de l'utilisateur alors qu'ils sont disposés sur les surfaces nasales (16) et comprenant une pluralité de moyens de communication (64) qui communiquent des informations à l'utilisateur liées à des informations physiologiques détectées par une ou plusieurs vibrations acoustiques, contacts tactiles ou émissions lumineuses.

2. Dispositif nasal (10) selon la revendication 1, **caractérisé en ce que** le dispositif de signal (22) est disposé au niveau de la première zone d'extrémité, de la seconde zone d'extrémité, ou de la zone intermédiaire.

3. Dispositif nasal (10) selon la revendication 1, **caractérisé en ce que** le dispositif de signal (22) reçoit des informations d'une source extérieure et utilise ces informations pour modifier un processus d'émission.

4. Dispositif nasal (10) selon la revendication 1, **caractérisé en ce que** le dispositif de signal (22) transmet les informations à un émetteur-récepteur distant pour un traitement successif du signal.

5. Dispositif nasal (10) selon la revendication 4, **caractérisé en ce que** le dispositif de signal (22) transmet par radiofréquence ou communication infrarouge.

6. Dispositif nasal (10) selon la revendication 1, **caractérisé en ce que** le dispositif de signal (22) comprend un moyen de mémoire permettant de mémoriser les informations.

7. Dispositif nasal (10) selon la revendication 1, **caractérisé en ce que** le dispositif de signal (22) comprend un moyen de temporisation pour contrôler l'application d'un contact tactile.

8. Dispositif nasal (10) selon la revendication 7, **caractérisé en ce que** le contact tactile est une vibration périodique.

9. Dispositif nasal (10) selon la revendication 1, **caractérisé en ce que** le dispositif de signal (22) est intégré dans des bandes flexibles (12) de matériau.

10. Dispositif nasal (10) selon la revendication 1, **caractérisé en ce qu'**il comprend en outre un élément résilient couplé à la bande flexible (12) qui fournit une force de levage à des parties des surfaces nasales (16) de l'utilisateur.

11. Dispositif nasal (10) selon la revendication 1, **caractérisé par** une couche adhésive (14) couplée à la bande flexible (12) de matériau, pour fixer ladite bande flexible aux surfaces nasales (16) et un moyen de vibration tactile (66) pour communiquer des informations tactiles à un utilisateur au niveau des surfaces nasales (16), lesdites informations tactiles étant liées aux informations physiologiques.

12. Dispositif nasal (10) selon la revendication 11, **caractérisé en ce que** le moyen de capteur comprend un capteur acoustique pour détecter les vibrations des surfaces nasales (16) liées au schéma de respiration de l'utilisateur.

13. Dispositif nasal (10) selon la revendication 11, **caractérisé par** un élément résilient couplé à la bande flexible de matériau qui fournit une force de levage aux surfaces nasales (16) de l'utilisateur.

14. Dispositif nasal (10) selon la revendication 11, **caractérisé en ce que** le moyen de vibration tactile (66) fournit une transmission tactile périodique aux surfaces nasales (16) de l'utilisateur.

15. Dispositif nasal (10) selon la revendication 14, **caractérisé en ce qu'**il comprend un microprocesseur (60) pour recevoir un signal d'un capteur (58, 60, 62) et pour contrôler une fonction du moyen de vibration tactile (66).
